# EUROPEAN PATENT APPLICATION

(11) **EP 1 174 149 A1**
(43) Date of publication of application: **23.01.2002**
(21) Application number: 00917362.6
(22) Date of filing: 19.04.2000
(51) Int. Cl.: A61K 45/00, A61K 38/00, A61K 39/395, A61K 48/00, A61K 31/7105, A61K 31/711, A61P 35/00, A61P 43/00, C12Q 1/68, G01N 33/60, G01N 33/15, G01N 33/53

(54) **PROLIFERATION INHIBITOR FOR ANDROGEN-INDEPENDENT TUMOR**

(30) Priority: 19.04.1999 JP 11102699
(71) Applicant: KYOWA HAKKO KOGYO CO., LTD., Chiyoda-ku, Tokyo 100-8185 (JP)
(72) Inventor: TANAKA, Akira, Kawachi-gun, Tochigi 329-0434 (JP)
(74) Representative: VOSSIUS & PARTNER
(86) International application number: JP0002544
(87) International publication number: WO0062809

(57) **Abstract**

The present invention relates to an inhibitor of the growth of an androgen-independent tumor, agents for treating and diagnosing an androgen-independent tumor, a method for diagnosing an androgen-independent tumor, and a method for screening a substance which inhibits the growth of an androgen-independent tumor.

## Description

### TECHNICAL FIELD

The present invention relates to an inhibitor of the growth of an androgen-independent tumor, agents for treating and diagnosing an androgen-independent tumor, a method for diagnosing an androgen-independent tumor, and a method for screening a substance which inhibits the growth of an androgen-independent tumor.

### BACKGROUND ART

Prostatic cancer is ranked second in Europe and the U.S. as the mortality rate of malignant tumors in adult men and increasing every year in Japan too, and its frequency is high (H. Akaza, *Can to Kagaku Ryoho,* 23, 401 (1996)). It is known that prostatic cancer has a particular biological character, androgen-dependency. Thus, an anti-androgen therapy is the main therapeutic method for prostatic cancer patients, and this therapeutic method is effective in 80 to 90% of prostatic cancer patients.

However, the most serious problem with the anti-androgen therapy is that the therapy ultimately becomes ineffective after continued treatment. In most cases, the patients become androgen-independent several months to several years after commencement of the anti-androgen therapy and die of cancer within 6 to 18 months once fall into androgen-independent.

Since there is no effective therapeutic method for the androgen-independent tumors (also called hormone-refractory cancer), the development of an effective therapeutic method has been strongly desired (H. Akaza, *Gan to Kagaku Ryoho*, 23, 401 (1996); Y. Hirao, *Gan* to *Kagaku Ryoho*, 23, 418 (1996)). Also, great concern has been directed toward the development of a method for precisely diagnosing whether it is an androgen-dependent tumor or an androgen-independent tumor, and the development of a diagnostic method for monitoring changes from androgen-dependency to androgen-independency.

Although the mechanism for the induction of an androgen-independent tumor from an androgen-dependent tumor has hardly been elucidated, its analysis has been attempted using model experimental systems described below.

Since a mouse breast cancer Shionogi carcinoma 115 can grow androgen-dependently in the presence of physiological concentration of androgen, it is suitable as an experimental model of hormone-dependent breast cancer and prostatic cancer (Koga, M. *et al*., *Journal of Steroid Biochemistry and Molecular Biology,* 54, 1 (1995)). A member of the fibroblast growth factor (hereinafter referred to as "FGF") family, FGF-8, has been isolated from an androgen-dependent mouse breast cancer cell SC-3 derived from Shionogi carcinoma 115 tumor, and it has been found that FGF-8 is an autocrine growth factor of the androgen-dependent mouse breast cancer cell SC-3 (Tanaka, A. *et al*., *Proceeding of National Academy of Science USA*, 89, 8928 (1992)). The autocrine growth factor is a substance which acts upon a cell itself that produces the growth factor. Since FGF-8 is detected at a high frequency in human prostatic cancer tissues by immunohistochemical staining of clinical tissue samples using anti-FGF-8 antibody, involvement of FGF-8 in a hormone-dependent tumorigenesis of human prostate has been suggested (Tanaka, A. *et al., Cancer Research*, 58, 2053 (1998)). It has been reported that an androgen-independent tumor was developed when androgen was removed from the androgen-dependent mouse breast cancer cell Shionogi carcinoma 115 (Kitamura, Y. *et al*., *Cancer Research,* 39, 4713 (1979)). In addition, a mouse breast cancer cell CADO21 (Noguchi, S. *et al., Journal of Steroid Biochemistry,* 32, 479 (1989)) and an androgen-independent mouse breast cancer cell SC-4 (Nomura, N. *et al*., *Cancer Research,* 48, 4904 (1988)) have been established from the androgen-independent tumor. However, growth factors produced by the androgen-independent mouse breast cancer cells SC-4 and CADO21 have not been elucidated.

As described above, growth factors related to the growth of androgen-independent tumors have not been elucidated, and diagnostic and therapeutic methods effective for androgen-independent tumors have not been established.

Activin has been discovered as a factor which acts upon the pituitary gland and enhances secretion of follicle-stimulating hormone (FSH) (Vale, W. *et al*., *Nature,* 231, 776 (1986)). Activin comprises a dimer of 14 kilodalton activin β subunits, which is a growth factor belonging to the TGF-β super family for its primary structural homology. The activin β subunit exists in two types, βA and βB, and three dimer forms, βAβA, βBβB and βAβB , have been reported and named activin A (βAβA), activin B (βBβB) and activin AB (βAβB), respectively (Vale, W. et al., *Peptide growth factors and their receptors II,* Editor Sporn, M.B. & Roberts, A.B., published by Springer (1990)).

Promotion of pituitary FSH secretion, induction of erythroblast differentiation, osteogenesis, neuronal cell survival, promotion of insulin production and the like have been reported as functions of activin (Vale, W. *et al*., *Peptide growth factors and their receptors II,* Editor Sporn, M.B. & Roberts, A.B., published by Springer (1990)), but its growth-promoting activity of androgen-independent tumors is not known.

Follistatin is an FSH secretion inhibiting factor isolated from an mammal ovary (Ueno, N. *et al*., *Proceeding* *of National Academy of Science USA*, 84, 8282 (1987)). It has been reported that follistatin shows the affinity for activin (Nakamura, T. *et al., Science,* 247, 836 (1990)) and shows activity of neutralizing the activin activity (Kogawa, K. *et al., Endocrinology,* 128, 1434 (1991)). However, it is not known that it has an inhibitory activity on the growth of an androgen-independent tumor.

### DISCLOSURE OF THE INVENTION

Identification of a growth factor relating to the growth of an androgen-independent tumor, an androgen-independent tumor growth inhibitor which inhibits the growth, and agents for effectively diagnosing and treating an androgen-independent tumor are desired. Furthermore, a method which can accurately diagnose an androgen-dependent tumor and an androgen-independent tumor and a diagnostic method for monitoring the conversion of an androgen-dependent cell into an androgen-independent cell are also desired.

The present inventors have found that androgen-independent mouse breast cancer cells SC-4 and CADO21 derived from androgen-dependent mouse breast cancer cells produce activin which is a growth factor that promotes the growth of a fibroblast, NIH3T3, and the growth of the androgen-independent mouse breast cancer cell SC-4. Furthermore, they have found that among three types of activin (activin A: βAβA, activin B: βBβB, and activin AB: βAβB), activin B and a mixture of activin A and activin B particularly promote the growth of the androgen-independent mouse breast cancer cell SC-4. Moreover, they have found that since follistatin, which is an activin inhibitor, inhibits a growth-promoting factor produced by androgen-independent breast cancer cells, follistatin can be used as an inhibitor of the growth of an androgen-independent tumor. Thus, the present invention has been completed.

In addition, the present inventors have found that FGF-8, known as an autocrine growth factor of an androgen-dependent tumor, shows growth-promoting activity for not only an androgen-dependent tumor but also an androgen-independent tumor. Also, they have found that activin shows activity of promoting the growth of an androgen-independent tumor in cooperation with FGF-8. Thus, the present invention has been accomplished.

The present invention relates to an inhibitor of the growth of an androgen-independent cancer cell, and diagnosis or treatment of an androgen-independent tumor. That is, the present invention relates to the following (1) to (27).

(1) An inhibitor of the growth of an androgen-independent tumor, comprising, as an active ingredient, a substance which inhibits the growth of an androgen-independent tumor.

Examples of androgen-independent tumors include prostatic cancer, breast cancer and the like.

(2) The inhibitor of the growth of an androgen-independent tumor according to (1), wherein the substance which inhibits the growth of an androgen-independent tumor is at least one substance selected from the group consisting of a substance having inhibitory activity for activin and a substance having inhibitory activity for fibroblast growth factor-8 (FGF-8).

Any substance having inhibitory activity for activin may be used, so long as it can inhibit growth promotion of an androgen-independent tumor by activin. Examples include an anti-activin neutralizing antibody (DePaolo, L.V. *et al*., *Endocrinology,* 130, 1741 (1991)), an anti-activin receptor neutralizing antibody, an activin binding protein such as follistatin (Nakamura, T. *et al., Science,* 247, 836 (1990); Kogawa, *K. et al., Endocrinology,* 128, 1434 (1991)) and the like, an antisense RNA/DNA of activin (Demura, R *et al*., *Endocrine Journal,* 43, 403 (1996)), a low molecular weight compound capable of inhibiting the expression of activin, a low molecular weight compound capable of inhibiting the function of activin by binding to activin, an antagonist of activin, derivatives thereof, and the like. Follistatin is preferably used.

Examples of substances having inhibitory activity for FGF-8 include an anti-FGF-8 neutralizing antibody (Japanese Published Unexamined Patent Application No. 271391-97; Tanaka A. *et al*., *Cancer Research*, 58, 2053 (1998)), an anti-FGF-8 receptor neutralizing antibody, an antisense RNA/DNA of FGF-8, a low molecular weight compound capable of inhibiting the expression of FGF-8, a low molecular weight compound capable of inhibiting the function of FGF-8 by binding to FGF-8, an antagonist of FGF-8, derivatives thereof, and the like. An anti-FGF-8 neutralizing antibody, particularly anti-FGF-8 neutralizing antibody KM1334 (FERM BP-5451), is preferably used.

The above substances having inhibitory activity for activin and substances having inhibitory activity for FGF-8 can inhibit the growth of an androgen-independent tumor by a single substance, but the growth of an androgen-independent tumor can be inhibited more effectively when a substance having inhibitory activity for activin and a substance having inhibitory activity for FGF-8 are jointly used.

(3) The inhibitor of the growth of an androgen-independent tumor according to (1) or (2), wherein the substance which inhibits the growth of an androgen-independent tumor is a substance having inhibitory activity for activin.

(4) The inhibitor of the growth of an androgen-independent tumor according to (2) or (3), wherein the substance having inhibitory activity for activin is a substance selected from the group consisting of an anti-activin neutralizing antibody, an anti-activin receptor neutralizing antibody, an activin binding protein and an antisense DNA/RNA of activin.

(5) The inhibitor of the growth of an androgen-independent tumor according to (4), wherein the activin binding protein is follistatin.

(6) The inhibitor of the growth of an androgen-independent tumor according to (1) or (2), wherein the substance which inhibits the growth of an androgen-independent tumor is a substance having inhibitory activity for fibroblast growth factor-8 (FGF-8).

(7) The inhibitor of the growth of an androgen-independent tumor according to (2) or (6), wherein the substance having inhibitory activity for fibroblast growth factor-8 (FGF-8) is an anti-FGF-8 neutralizing antibody.

(8) An agent for treating an androgen-independent tumor, comprising, as an active ingredient, a substance which inhibits the growth of an androgen-independent tumor.

(9) The agent for treating an androgen-independent tumor according to (8), wherein the substance which inhibits the growth of an androgen-independent tumor is at least one substance selected from the group consisting of a substance having inhibitory activity for activin and a substance having inhibitory activity for fibroblast growth factor-8 (FGF-8).

(10) The agent for treating an androgen-independent tumor according to (8) or (9), wherein the substance which inhibits the growth of an androgen-independent tumor is a substance having inhibitory activity for activin.

(11) The agent for treating an androgen-independent tumor according to (9) or (10), wherein the substance having inhibitory activity for activin is a substance selected from the group consisting of an anti-activin neutralizing antibody, an anti-activin receptor neutralizing antibody, an activin binding protein and an antisense DNA/RNA of activin.

(12) The agent for treating an androgen-independent tumor according to (11), wherein the activin binding protein is follistatin.

(13) The agent for treating an androgen-independent tumor according to (8) or (9), wherein the substance which inhibits the growth of an androgen-independent tumor is a substance having inhibitory activity for fibroblast growth factor-8 (FGF-8).

(14) The agent for treating an androgen-independent tumor according to (7) or (13), wherein the substance having inhibitory activity for fibroblast growth factor-8 (FGF-8) is a substance selected from the group consisting of an anti-FGF-8 neutralizing antibody, an anti-FGF-8 receptor neutralizing antibody and an antisense DNA/RNA of FGF-8.

(15) A method for detecting activin, comprising using a substance selected from the group consisting of an anti-activin antibody, an activin binding protein and a DNA/RNA coding for activin.

(16) The method for detecting activin according to (15), wherein the activin binding protein is follistatin.

(17) A method for determining activin, comprising using a substance selected from the group consisting of an anti-activin antibody, an activin binding protein and a DNA/RNA coding for activin.

(18) The method for determining activin according to (16), wherein the activin binding protein is follistatin.

(19) A method for diagnosing an androgen-independent tumor, comprising using a substance selected from the group consisting of an anti-activin antibody, an activin binding protein, a DNA/RNA coding for activin, an anti-FGF-8 antibody and a DNA/RNA coding for FGF-8.

(20) The method for diagnosing an androgen-independent tumor according to (19), wherein the activin binding protein is follistatin.

(21) A method for diagnosing the conversion of an androgen-dependent cell into an androgen-independent cell, comprising using a substance selected from the group consisting of an anti-activin antibody, an activin binding protein, a DNA/RNA coding for activin, an anti-FGF-8 antibody and a DNA/RNA coding for FGF-8.

(22) The method according to (21), wherein the activin binding protein is follistatin.

(23) An agent for diagnosing an androgen-independent tumor, comprising, as an active ingredient, a substance selected from the group consisting of an anti-activin antibody, an activin binding protein, a DNA/RNA coding for activin, an anti-FGF-8 antibody and a DNA/RNA coding for FGF-8.

(24) The agent for diagnosing an androgen-independent tumor according to (23), wherein the activin binding protein is follistatin.

(25) A method for screening an inhibitor of a growth factor for an androgen-independent tumor, comprising using a growth factor for an androgen-independent tumor.

A substance which inhibits growth-promoting activity for androgen-independent tumor cells can be screened by using a growth factor for an androgen-independent tumor found in the present invention. Activin, FGF-8 and the like can be used as suitable examples of the growth factor for an androgen-independent tumor.

The screening method includes MTT method, ³H-TdR incorporation method and the like described later. By this method, an agent capable of inhibiting the growth of an androgen-independent cell can be screened. The substance obtained by the screening is useful as an agent for treating an androgen-independent tumor cell.

(26) The method for screening an inhibitor of a growth factor for an androgen-independent tumor according to (25), wherein the growth factor for an androgen-independent tumor is activin.

(27) The method for screening an inhibitor of a growth factor for an androgen-independent tumor according to (25), wherein the growth factor for androgen-independent tumors is fibroblast growth factor-8 (FGF-8).

### 1. Isolation and purification of growth factor for androgen-independent tumor

A growth factor for an androgen-independent tumor can be isolated by the following method.

Any androgen-independent tumor may be used, so long as it is a cell derived from an androgen-dependent cell and can grow androgen-independently. Examples include an androgen-independent mouse breast cancer cell SC-4, an androgen-independent mouse breast cancer cell CADO21, androgen-independent human prostatic cancer cells DU145 (ATCC HTB-81) and PC3 (ATCC CRL-1435), and the like.

A growth factor for an androgen-independent tumor produced by the androgen-independent tumor can be obtained from a culture obtained by culturing a tumor cell in an appropriate medium or from a lysate of the resulting cell.

The growth factor for androgen-independent tumor can be isolated and purified by separation techniques, such as solvent extraction, organic solvent fractional precipitation, salting out, centrifugation, ultrafiltration, ion exchange chromatography, gel filtration chromatography, hydrophobic chromatography, affinity chromatography, reverse phase chromatography, crystallization, electrophoresis and the like, alone or in combination. The growth factor for androgen-independent tumor cells can be identified by adding the growth factor to an androgen-independent cells, culturing the resulting cells and then measuring the number of proliferated cells by the MTT method (Tanaka, A. *et al*., *Cancer Research,* 58, 2053 (1988)), the ³H-TdR incorporation method (Tanaka, *A*., *et al*., *Proceeding of National Academy of Science USA*, 89, 8928 (1992)) or the like.

Although activin (Vale, W. *et al*., *Nature,* 321, *776* (1986); Ling, N. *et al*., *Nature,* 321, 779 (1986)) can be obtained by the above method as a growth factor for an androgen-independent tumor produced by the androgen-independent tumor, activin can also be obtained using known genetic engineering techniques (*Molecular Cloning,* Second Edition) and the like.

### 2. Agent for diagnosing and treating androgen-independent tumors

A medicament comprising the substance of the present invention capable of inhibiting activin activity can be administered as a therapeutic agent alone, but generally, it is preferable to provide it as a pharmaceutical formulation produced by an appropriate method well known in the technical field of pharmaceutics, by mixing it with one or more pharmaceutically acceptable carriers.

It is preferable to use a route of administration which is most effective in treatment. Examples include oral administration and parenteral administration, such as buccal, airway, rectal, subcutaneous, intramuscular, intravenous administration and the like. Intravenous administration is preferred in an antibody-containing pharmaceutical formulation.

The dosage form includes sprays, capsules, tablets, granules, syrups, emulsions, suppositories, injections, ointments, tapes and the like.

Pharmaceutical formulations suitable for oral administration include emulsions, syrups, capsules, tablets, powders, granules and the like.

Liquid formulations such as emulsions and syrups can be produced using, as additives, water; saccharides, such as sucrose, sorbitol, fructose, *etc.*; glycols, such as polyethylene glycol, propylene glycol, *etc.*; oils such as sesame oil, olive oil, soybean oil, *etc.*; antiseptics such as p-hydroxybenzoic acid esters, *etc.*; flavors, such as strawberry flavor, peppermint, *etc.*; and the like.

Capsules, tablets, powders, granules and the like can be produced using, as additives, fillers, such as lactose, glucose, sucrose, mannitol, *etc.*; disintegrating agent, such as starch, sodium alginate, *etc.*; lubricants, such as magnesium stearate, talc, *etc.*; binders, such as polyvinyl alcohol, hydroxypropylcellulose, gelatin, *etc.;* surfactants, such as fatty acid ester, *etc.;* plasticizers, such as glycerol, *etc.;* and the like.

Examples of the pharmaceutical formulation suitable for parenteral administration include injections, suppositories, sprays and the like.

Injections are prepared using a carrier, such as a salt solution, a glucose solution or a mixture of both of them, or the like. Alternatively, powder injections can be prepared by freeze-drying a protein, a peptide or an antibody in the usual way and then adding sodium chloride thereto.

Suppositories are prepared using a carrier such as cacao butter or a hydrogenated fat or carboxylic acid.

Also, sprays are prepared using the compound as such or using a carrier which does not stimulate the buccal or airway mucous membrane of the patient and can facilitate absorption of the compound by dispersing it as fine particles.

Examples of the carrier include lactose, glycerol and the like. Depending on the properties of the compound and the carrier to be used, it is possible to produce a pharmaceutical formulation, such as aerosols, dry powders or the like. In addition, the components exemplified as additives for oral preparations can also be added to the parenteral formulation.

Although the clinical dose or the frequency of administration varies depending on the intended therapeutic effect, administration method, treating period, age, body weight and the like, it is usually from 10 µg/kg to 20 mg/kg per day per adult.

### 3. Method for diagnosing androgen-independent tumors

It is useful for the diagnosis of an androgen-independent tumor to detect or determine activin which is a growth factor produced by an androgen-independent tumor found in the present invention. As the detection or determination method of activin, the following methods and the like can be employed, which use an anti-activin antibody or an activin binding protein such as follistatin.

The method for detecting or determining activin in a cell or tissue includes an immunofluorescent method, enzyme-linked immunosorbent assay (ELISA), radioimmunoassay (RIA), immunohistochemistry (ABC method, CSA method, *etc.*), such as tissue immunostaining, cell immunostaining, *etc.,* Western blotting, dot blotting, immunoprecipitation (*Monoclonal Antibody Laboratory Manual* (Kodan-sha Scientific, 1987), Second Series Biochemical Experimentation Course 5 Immunobiochemical Method (Tokyo Kagaku Dojin, 1986)) and the like.

The immunofluorescent method is a method in which activin existing in a cell or tissue is allowed to react with an anti-activin antibody or an activin binding protein, such as follistatin or the like, and further allowed to react with an antibody for the anti-activin antibody, an anti-follistatin antibody or a binding fragment thereof, labeled with a fluorescent material, such as fluorescein isothiocyanate (FITC) or the like, and then the fluorescent dye is measured using a flow cytometer.

Enzyme-linked immunosorbent assay (ELISA) is a method in which activin existing in a cell or tissue is allowed to react with an anti-activin antibody or an activin binding protein, such as follistatin or the like, and further allowed to react with an antibody for the anti-activin antibody, an anti-follistatin antibody or a binding fragment thereof, labeled with an enzyme, such as peroxidase, biotin or the like, and then the colored dye is measured using a spectrophotometer.

Radioimmunoassay (RIA) is a method in which activin existing in a cell or tissue is allowed to react with an anti-activin antibody or an activin binding protein, such as follistatin or the like, and further allowed to react with an antibody for the anti-activin antibody, an anti-follistatin antibody or a binding fragment thereof, labeled with a radioactive isotope, and then the radioactivity is measured using a scintillation counter or the like.

Cell immunostaining or tissue immunostaining is a method in which activin existing in a cell or tissue is allowed to react with an anti-activin antibody or an activin binding protein, such as follistatin or the like, and further allowed to react with an antibody for the anti-activin antibody, an anti-follistatin antibody or a binding fragment thereof labeled with a fluorescent material, such as FITC or the like, or an enzyme, such as peroxidase, biotin or the like, and then the sample is observed under a microscope.

Western blotting is a method in which activin existing in a cell or tissue is fractionated by SDS-polyacrylamide gel electrophoresis (*Antibodies-A Laboratory Manual,* Cold Spring Harbor Laboratory, 1988), the gel is blotted on a PVDF membrane or a nitrocellulose membrane, the membrane is allowed to react with an anti-activin antibody or an activin binding protein, such as follistatin or the like, and then with an antibody for the anti-activin antibody, an anti-follistatin antibody or a binding fragment thereof labeled with a fluorescent material, such as FITC or the like, or an enzyme, such as peroxidase, biotin or the like, and then activin existing in a cell or tissue is confirmed.

Dot blotting is a method in which activin existing in a cell or tissue is blotted on a nitrocellulose membrane, the membrane is allowed to react with an anti-activin antibody or an activin binding protein, such as follistatin or the like, and then with an antibody for the anti-activin antibody, an anti-follistatin antibody or a binding fragment thereof labeled with a fluorescent material, such as FITC or the like, or an enzyme, such as peroxidase, biotin or the like, and then activin existing in a cell or tissue is confirmed.

Immunoprecipitation is a method in which activin existing in a cell or tissue is allowed to react with an anti-activin antibody or an activin binding protein, such as follistatin or the like, and then mixed with a carrier, such as protein G-Sepharose or the like, having ability of specifically binding to the immunoglobulin, to thereby precipitate the antigen-antibody complex.

Also, activin in a tissue or cell can be detected or determined using DNA, RNA or an oligonucleotide encoding activin, by genetic engineering technique, such as Northern blotting (*New Cell Engineering Experimentation Protocols,* Shujun-sha, 1993), PCR (*PCR Protocols,* Academic Press, 1990), in situ hybridization (*In Situ Hybridization Techniques,* Gakusai Kikaku, 1992) or the like.

Northern blotting is a method in which mRNA or total RNA is extracted from a cell or tissue and fractionated by agarose gel electrophoresis (*Molecular Cloning,* Second Edition) or the like, the gel is blotted on a membrane, such as nitrocellulose or the like, the membrane is allowed to react with a full or partial length activin cDNA labeled with a radioactive isotope or the like, as a probe, and then mRNA of activin existing in the cell or tissue is confirmed by autoradiography or the like.

PCR is a method in which mRNA or total RNA is extracted from a cell or tissue and mixed with primers designed based on a nucleotide sequence corresponding to activin, as well as a polymerase and the like, and then activin cDNA fragments are amplified and confirmed by agarose gel electrophoresis or the like.

*In situ* hybridization is a method in which activin mRNA existing in cells or tissues is allowed to react with a full or partial length activin cDNA labeled with biotin or the like, as a probe, and further allowed to react with enzyme-labeled avidin or the like, and then the results are observed under a microscope.

In addition, since FGF-8 and activin have activity of promoting additional growth of an androgen-independent tumor and the growth factors produced by a hormone-dependent tumor and those produced by a hormone-independent tumor are different, conversion of an androgen-dependent tumor into an androgen-independent tumor can be monitored by detecting or determining FGF-8 and activin individually or simultaneously. Specifically, it can be carried out by allowing activin or FGF-8 existing in a cell or tissue to react with an anti-activin antibody or an activin binding protein, such as follistatin or the like, and with an anti-FGF-8 antibody, followed by detecting or determining by any of the above immunological methods.

Alternatively, activin and FGF-8 can be detected or determined individually or simultaneously, by the above genetic engineering techniques using DNA, RNA or an oligonucleotide encoding activin or FGF-8.

### BRIEF EXPLANATION OF THE DRAWINGS

Fig. 1 shows growth-promoting activity of culture supernatants of androgen-independent mouse breast cancer cells SC-4 and CADO21 on mouse fibroblast NIH3T3.

Fig. 2 shows growth-promoting activity of a fraction of a culture supernatant of an androgen-independent mouse breast cancer cell CADO21, purified by a heparin Sepharose column, on mouse fibroblast NIH3T3.

Fig. 3 shows growth-promoting activity of the fraction purified by a heparin column and purified activin on mouse fibroblast NIH3T3, and effect of follistatin to inhibit each growth-promoting activity.

Fig. 4 shows a result of the Western blotting detection of activin βA in a fraction purified by a copper-chelating column.

Fig. 5 shows growth-promoting activity of purified activin A, activin B, activin AB and a mixture of activin A with activin B, on mouse fibroblast NIH3T3.

Fig. 6 shows growth-promoting activity of purified activin A, activin B, activin AB and a mixture of activin A with activin B, on androgen-independent mouse breast cancer cell SC-4.

Fig. 7 shows growth-promoting activity of FGF-8 alone and purified activin A, activin B, activin AB and a mixture of activin A with activin B in the presence of FGF-8 respectively, on androgen-independent mouse breast cancer cell SC-4.

Fig. 8 shows a result of the RT-PCR detection of mRNA of activin βB, FGF-8 and G3PDH in androgen-non-stimulated mouse breast cancer cell SC-3 (lane 1), androgen-stimulated mouse breast cancer cell SC-3 (lane 2), androgen-non-stimulated mouse breast cancer cell SC-4 (lane 3), androgen-stimulated mouse breast cancer cell SC-4 (lane 4), androgen-non-stimulated mouse breast cancer cell CADO21 (lane 5) and androgen-stimulated mouse breast cancer cell CADO21 (lane 6).

### BEST MODE FOR CARRYING OUT THE INVENTION

### 1. Growth-promoting activity in culture supernatants of androgen-independent mouse breast cancer cells SC-4 and CADO21, for mouse fibroblast, NIH3T3 cell

In order to obtain autocrine and paracrine growth factors of androgen-independent mouse breast cancer cell SC-4 (Nonomura, N. *et al., Cancer Research*, 48, 4904 (1988)) and androgen-independent mouse breast cancer cell CADO21 (Noguchi, S. *et al., Journal of Steroid Biochemistry,* 32, 479 (1989)), growth factors produced by androgen-independent mouse breast cancer cells SC-4 and CADO21 were analyzed in the following manner using the growth-promoting activity for a mouse fibroblast cell line, NIH3T3 cell (Tanaka, A. *et al*., *FEBS Letters,* 363, 226 (1995)) as a marker. The paracrine growth factor is a substance which acts upon different cells adjacent to or in the vicinity of a growth factor-producing cell.

Each of 0.5×10⁶ cells of an androgen-independent mouse breast cancer cell SC-4, and 0.8×10⁶ cells of an androgen-independent mouse breast cancer cell CADO21, was suspended in DMEM:Ham's F-12 (1:1 v/v, manufactured by Nissui Pharmaceutical) medium containing 2% dextran charcoal-treated fetal bovine serum (FBS), added to a 100-mm dish, and cultured at 37°C in a CO₂ incubator. On the next day, the culture medium was changed to DMEM:Ham's F-12 (1:1 v/v, manufactured by Nissui Pharmaceutical) medium containing no FBS. Forty-eight hours after further culturing, 2 liters of each of the culture supernatants was recovered.

### Evaluation of NIH3T3 growth-promoting activity

NIH3T3 cells were suspended in DMEM:Ham's F-12 (1:1 v/v, manufactured by Nissui Pharmaceutical) medium containing 2% dextran charcoal-treated FBS, dispensed in 3×10³ cells/100 µl portions into each well of a 96-well plate, and then cultured. On the next day, the culture medium was removed, 100 µl of 1% FBS-added DMEM:Ham's F-12 (1:1 v/v, manufactured by Nissui Pharmaceutical) medium containing a sample to be tested was added, followed by culturing. Two days after culturing, the culture medium was removed, 100 µl of 1% FBS-added DMEM:Ham's F-12 (1:1 v/v, manufactured by Nissui Pharmaceutical) medium was added, followed by culturing. Two days thereafter, the number of cells in the culture was measured by MTT assay in accordance with the method described in a reference (Tanaka, A. *et al*., *Cancer Research,* 58, 2053 (1998)).

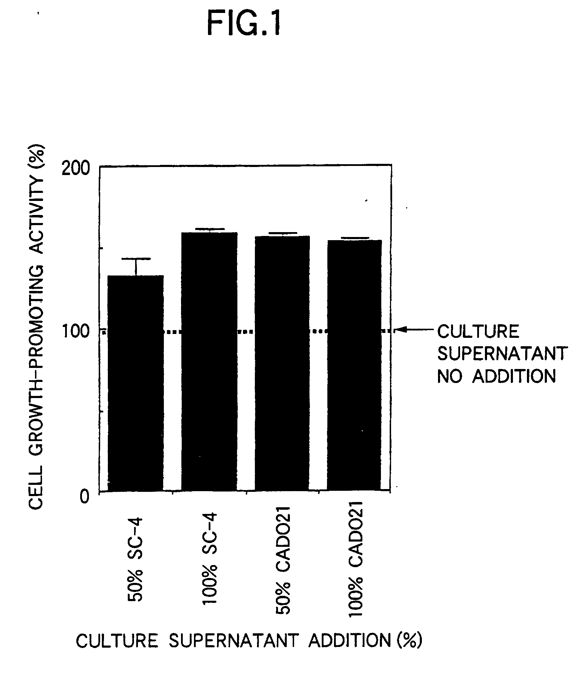

The results are shown in Fig. 1. When the culture supernatant of the above androgen-independent mouse breast cancer cell SC-4, was added in an amount of 50% or 100% as the sample to be tested, the growth-promoting activity for NIH3T3 was 132.5 ± 11.3% and 158.8 ± 3.1%, respectively. Also, when the culture supernatant of the androgen-independent mouse breast cancer cell CADO21, was added in an amount of 50% or 100%, the growth-promoting activity for NIH3T3 was 156.3 ± 2.5% and 153.1 ± 2.5%, respectively. Thus, the growth-promoting activity for NIH3T3 was found in the culture supernatant of the androgen-independent cells.

### 2. Purification of growth-promoting factor for mouse fibroblast, NIH3T3 cell, in culture supernatant of androgen-independent mouse breast cancer cell SC-4

The culture supernatant of the androgen-independent mouse breast cancer cell SC-4, and the culture supernatant of androgen-independent mouse breast cancer cell CADO21, shown in the above item 1 were concentrated using Pellicon-Labocassette (manufactured by Millipore) capable of removing molecules of 10 kilodaltons or less in molecular weight, in accordance with the method described in a reference (Tanaka, A. *et al*., *Proceeding of National Academy of Science USA*, 89, 8298 (1992)). In accordance with the method described in the reference (Tanaka, A. *et al*., *Proceeding of National Academy of Science USA*, 89, 8298 (1992)), the thus concentrated culture supernatant was applied to a heparin Sepharose column (gel volume 1 ml: manufactured by Amersham Pharmacia Biotech) which had been equilibrated with 10 mM Tris/HCl buffer (pH 7.5) containing 0.1 M NaCl, 0.1% CHAPS {3-[(3-cholamidopropyl)dimethylammonio]-1-propanesulfonate, manufactured by Wako Pure Chemical Industries}, 2 mM phenylmethylsulfonyl fluoride and 200 µg/ml of leupeptin (hereinafter referred to as "equilibration buffer").

After washing the column with the above equilibration buffer, 10 ml of each of equilibration buffers containing 0.3 M, 0.6 M and 2 M NaCl was passed through the column, and the protein adsorbed to the column was successively eluted in 1 ml into a total of 10 tubes. The activity of the heparin column-eluted fractions was evaluated by adding 2% of each of the eluted fractions (each of the first 5 tubes) as the sample to be tested, to the NIH3T3 cell-used growth assay shown in the above item 1.

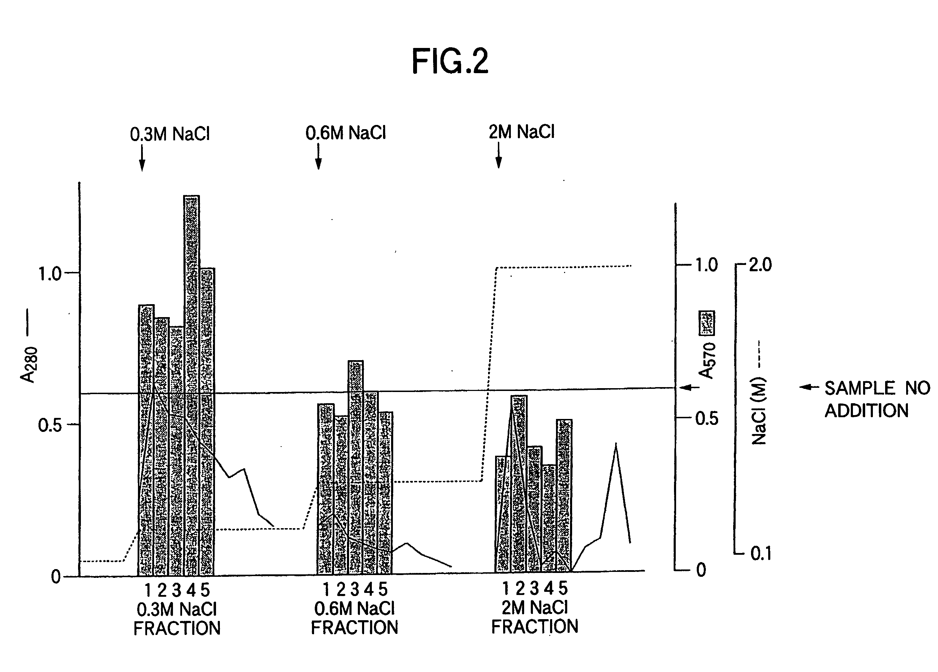

A result of the purification of the culture supernatant of the androgen-independent mouse breast cancer cell CADO21 is shown in Fig. 2. The growth-promoting factor of NIH3T3 cell was recovered in the 0.3 M NaCl-eluted fraction. Regarding the culture supernatant of the androgen-independent mouse breast cancer cell SC-4, the growth-promoting factor of NIH3T3 cell was recovered also in the 0.3 M NaCl-eluted fraction as shown in Table 1.

**Table 1**

| Sample | NIH3T3 cell growth activity |
|---|---|
| SC-4 culture supernatant | + |
| Heparin column | |
| 0.3 M NaCl-eluted fraction | + |
| 0.6 M NaCl-eluted fraction | - |
| 2 M NaCl-eluted fraction | - |

Regarding the androgen-independent mouse breast cancer cell CADO21 culture supernatant, the 0.3 M NaCl-eluted fraction was recovered and applied to a copper-chelating column (gel volume 0.8 ml: manufactured by Pharmacia) which had been equilibrated with the above equilibration buffer. After washing the column with the equilibration buffer, 5 ml of each of equilibration buffers containing 2 mM, 5 mM, 10 mM and 20 mM imidazole was passed through the column, and the protein adsorbed to the column was successively eluted. The activity of the eluted fractions was evaluated by adding 2% of each eluted fraction as the sample to be tested, to the NIH3T3 cell-used growth assay shown in the above item 1.

The results are shown in Table 2. The NIH3T3 cell growth-promoting activity was recovered in the 10 mM imidazole-eluted fraction.

**Table 2**

| Sample | NIH3T3 cell growth activity |
|---|---|
| Heparin column | |
| 0.3 M MaCl-eluted fraction | + |
| Copper-chelating column | |
| 2 mM imidazole-eluted fraction | - |
| 5 mM imidazole-eluted fraction | + |
| 10 mM imidazole-eluted fraction | - |
| 20 mM imidazole-eluted fraction | - |

### 3. Identification of NIH3T3 cell growth-promoting factor in culture supernatant of androgen-independent mouse breast cancer cell SC-4

The growth-promoting factor in the culture supernatants of androgen-independent mouse breast cancer cells SC-4 and CADO21 was eluted with 0.3 M NaCl by the heparin column-used purification carried out in the above item 2. In general, growth factors of the FGF family have high affinity for heparin (Burgess, W.H. *et al*., *Annual Review of Biochemistry,* 58, 575 (1989)), but it was considered that the eluted growth-promoting factor does not belong to the growth factors of FGF family because of its markedly weak affinity for heparin.

In addition, since the above factor has a growth-promoting activity for the fibroblast NIH3T3 cell, it was considered to be a factor belonging to the TGF (transforming growth factor)-β super family. The following analysis was carried out aiming at activin (Munier, H. *et* *al*., *Proceeding of National Academy of Science USA*, 85, 247-251 (1988)) which is frequently expressed in endocrine organs among the TGF-β super family.

The heparin column-purified fraction of the culture supernatant of the androgen-independent mouse breast cancer cell CADO21 having activity of promoting the growth of NIH3T3 cell, obtained in the above item 2, was added to the NIH3T3 cell growth assay shown in the above item 1 to give a concentration of 1%, and human follistatin (received from Dr. Palow at the National Hormone & Pituitary Program, USA: *Journal of Endocrinology,* 154, 535 (1997)) was further added thereto to give a final concentration of 10 nM. Follistatin is an activin binding protein and has ability of neutralizing the activin activity.

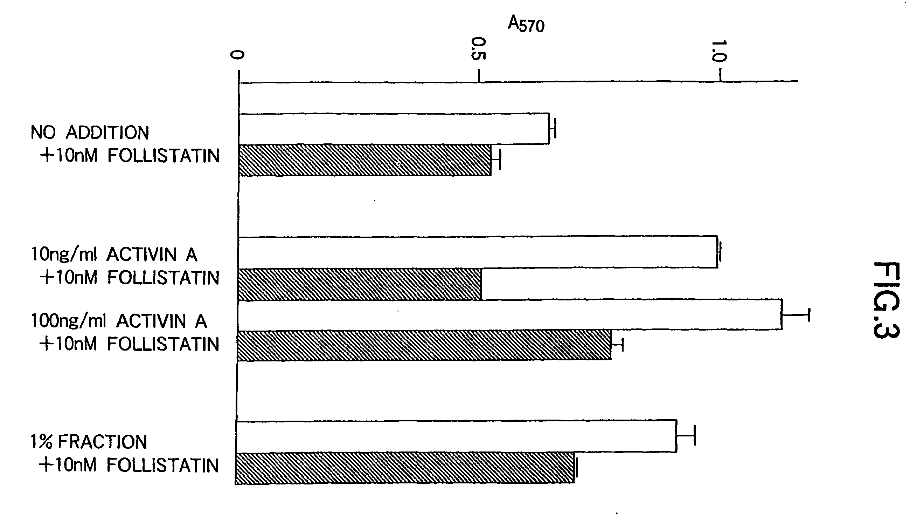

The results are shown in Fig. 3. Human follistatin did not show its effect on the growth of NIH3T3 cell at a final concentration of 10 nM, but showed inhibitory activity for the NIH3T3 cell growth-promoting activity in the heparin column-purified fraction. Also, it was confirmed that the bovine activin A (β chain homodimer: βAβA) (Hasegawa, Y. *et al*., *Hormone Research*, 41 (supplement 1), 55 (1994)) used as a positive control promotes the growth of NIH3T3 cell at concentrations of 10 and 100 ng/ml and that follistatin inhibits the activity of activin A at a concentration of 10 nM.

Since the NIH3T3 cell growth-promoting activity in the culture supernatant of the androgen-independent mouse breast cancer cell CADO21 was inhibited by follistatin, the presence of activin reported as a follistatin binding factor was suggested. Accordingly, it was examined whether activin exists or not in the NIH3T3 cell growth-promoting fraction purified using the copper-chelating column shown in the above item 2 in the following manner by Western blotting using an anti-activin βA monoclonal antibody.

In accordance with the method of a reference (Tanaka, A. *et al*., *Cancer Research,* 58, 2053 (1998)), SDS-PAGE was carried out as described below using 10 to 20% gradient polyacrylamide gel (manufactured by Daiichi Chemical).

A 1/10 volume of the fraction purified using the copper-chelating column obtained in the above item 2 was applied to a lane on the gel and subjected to electrophoresis. After the electrophoresis, the gel was transferred to a PVDF membrane (manufactured by Bio-Rad). The membrane was allowed to react with 100,000 times-diluted anti-activin βA monoclonal antibody (Miyamoto, K. *et al*., *BBRC,* 136, 1103-1109 (1986)), and activin βA was detected using ECL Plus detection kit (manufactured by Amersham) in accordance with the manufacture's instructions.

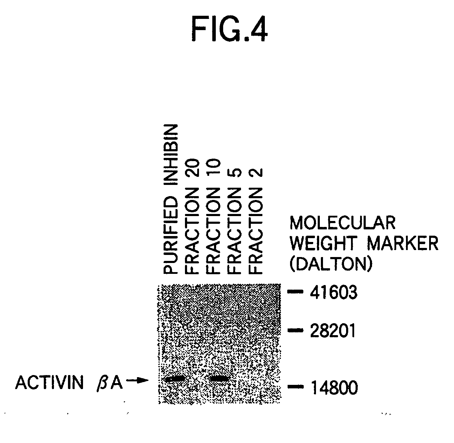

The results are shown in Fig. 4. A band corresponding to about 14.8 kilodaltons in molecular weight and reacting with the anti-activin βA monoclonal antibody was detected in the fraction purified with the copper-chelating column. Also, mobility of the band coincided with the band of activin βA in the positive control human recombinant inhibin A (heterodimer of activin βA chain (18 kilodaltons in molecular weight) and inhibin α (18 kilodaltons in molecular weight)). Thus, it was revealed that activin βA is contained in the fraction purified using the copper-chelating column.

In order to confirm that activin is a growth-promoting factor of NIH3T3 cell, the growth-promoting activity for NIH3T3 cell was examined by the method shown in the above item 1, using purified bovine activin A (β chain homodimer: βAβA), activin B (β chain homodimer: βBβB) and activin AB (β chain homodimer: βAβB) (Hasegawa, Y. *et al*., *Hormone Research,* 41 (supplement 1), 55 (1994)) and recombinant human inhibin A (α chain-(3 chain heterodimer: αAβB) (manufactured by National Hormone & Pituitary Program, USA).

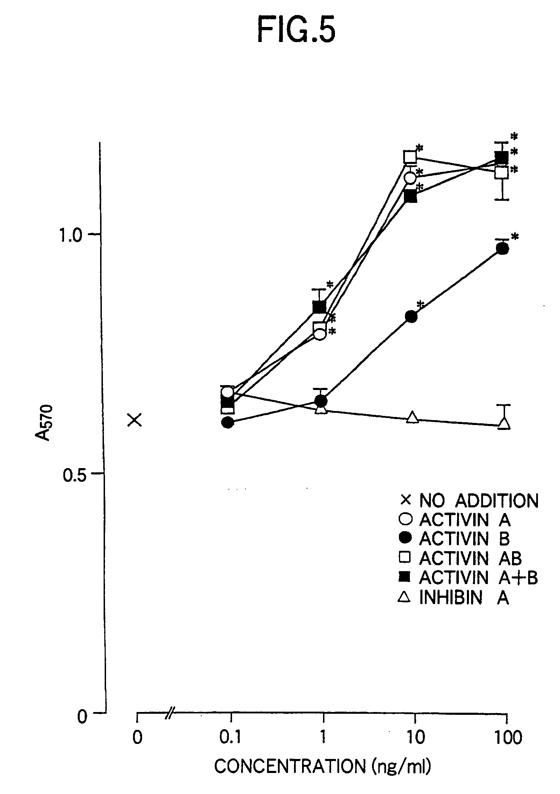

The results are shown in Fig. 5. All of the purified three activin species showed the NIH3T3 cell growth-promoting activity concentration-dependently. On the other hand, inhibin A showed no growth-promoting activity for NIH3T3 cell. Thus, it was revealed that the β chain homodimers (βAβA, βBβB and βAβB) specifically have the growth-promoting activity for NIH3T3 cell.

### 4. Growth-promoting activity of activin on androgen-independent mouse breast cancer cell SC-4

Activity of activin on the growth of androgen-independent mouse breast cancer cell SC-4, was examined using the purified bovine activin A, activin B and activin AB shown in the above item 3.

The androgen-independent mouse breast cancer cell SC-4 was suspended in DMEM:Ham's F-12 (1:1 v/v, manufactured by Nissui Pharmaceutical) medium containing 2% dextran charcoal-treated FBS, dispensed in 1.5x10³ cells/100 µl portions into each well of a 96-well plate and then cultured. On the next day, the culture medium was removed, 100 µl of 1% FBS-added DMEM:Ham's F-12 (1:1 v/v, manufactured by Nissui Pharmaceutical) medium containing a sample to be tested was added, followed by culturing. Two days thereafter, the culture medium was exchanged, followed by culturing for one day, and then the number of cells in the culture was measured by MTT assay in accordance with the method described in a reference (Tanaka, A. *et al., Cancer Research*, 58, 2053 (1998)).

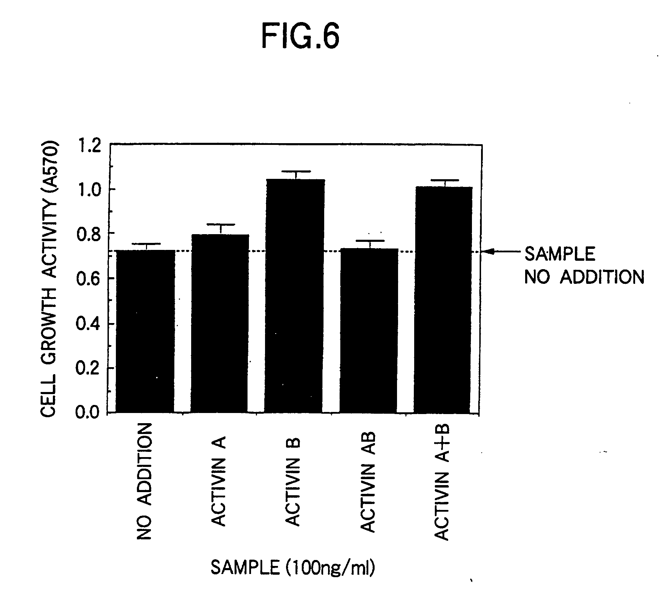

The results are shown in Fig. 6. When each of activin B and a mixture of activin A and activin B was added to give a concentration of 100 ng/ml, growth of the androgen-independent mouse breast cancer cell SC-4 became 145% and 138%, respectively, in comparison with the sample of no addition. On the other hand, the growths when activin A and activin AB were added became 110% and 100%, respectively, in comparison with the sample of no addition. Thus, it was revealed that, among the three activin species, activin B has the highest growth-promoting activity for the androgen-independent mouse breast cancer cell SC-4.

It has been reported that FGF-8 found as an autocrine growth factor of the androgen-dependent mouse breast cancer cell, Shionogi carcinoma 115, is important as a growth factor of an androgen-dependent tumor (Tanaka, A. *et al., Proceeding of National Academy of Science USA*, 89, 8928 (1992)), but its relation to an androgen-independent tumor is not known. Accordingly, effect of FGF-8 on the growth-promoting activity of purified activin B as a growth factor of androgen-independent cells was examined in the same manner as described above.

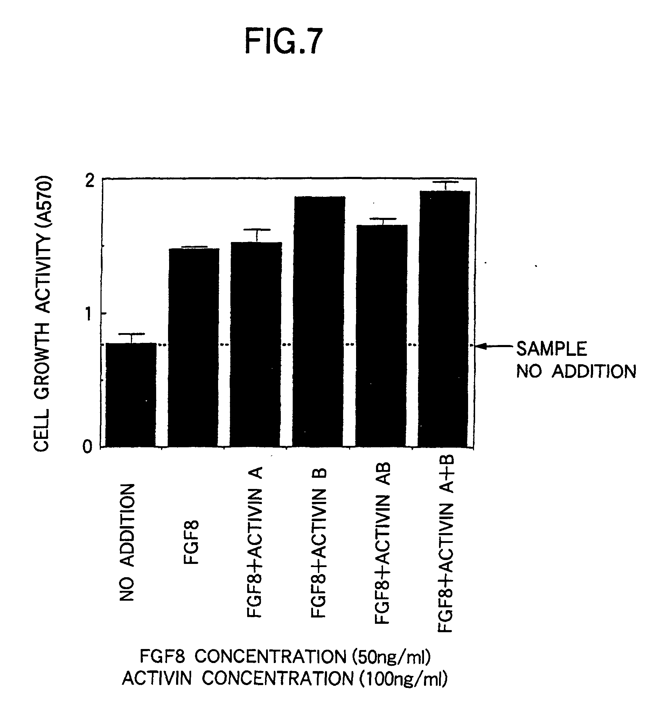

The results are shown in Fig. 7. Purified FGF-8 (Tanaka, A. *et al*., *FEBS Letters,* 363, 226 (1995)) showed the activity of promoting the growth of androgen-independent mouse breast cancer cell SC-4 alone in an amount of 50 ng/ml. Also, activin B and a mixture of activin A and activin B showed activity of further enhancing the activity of FGF-8 for promoting androgen-independent mouse breast cancer cell SC-4, at a concentration of 100 ng/ml. Activin A and activin AB show no activity of enhancing the growth-promoting activity by FGF-8.

Based on the above results, it was revealed that activin B is a growth-promoting factor of androgen-independent mouse breast cancer cell SC-4, that FGF-8 not only has the androgen-dependent cell growth activity but also has growth-promoting activity for the androgen-independent mouse breast cancer cell SC-4, and that the activity of FGF-8 for promoting the growth of the androgen-independent mouse breast cancer cell SC-4 is further enhanced by activin B.

### 5. Detection of FGF-8 and activin mRNA in androgen-dependent mouse breast cancer cell SC-3 and androgen-independent mouse breast cancer cells SC-4 and CADO21 by RT-PCR

Detection and alteration of expression level of FGF-8 and activin mRNA in androgen-dependent mouse breast cancer cell SC-3 and androgen-independent mouse breast cancer cells SC-4 and CADO21 were examined by RT-PCR.

Androgen stimulation to the androgen-dependent mouse breast cancer cell SC-3, and androgen-independent mouse breast cancer cells SC-4 and CADO21, was carried out in accordance with the method described in a reference (Tanaka, A. *et al*., *Proceeding of National Academy of Science USA*, 89, 8928 (1992)). A preparation of total RNA from cells and the RT-PCR were carried out in accordance with the method described in a reference (Tanaka, A. *et al., FEBS Letters*, 363, 226 (1995)). The RT-PCR was carried out for 48 hours by the above reference-described method and, regarding the primers used, the synthetic DNA fragments described in SEQ ID NOs:1 and 2 were used as a primer set for detecting mouse activin βB, and the synthetic DNA fragments described in SEQ ID NOs:3 and 4 as a primer set for detecting mouse FGF-8, respectively. A primer set for detecting mouse G3PDH (glyceraldehyde-3-phosphate dehydrogenase) gene which is a control gene generally used for the evaluation of expression quantity of genes (Mouse G3PDH Control Amplimer Set) was purchased from Clontech.

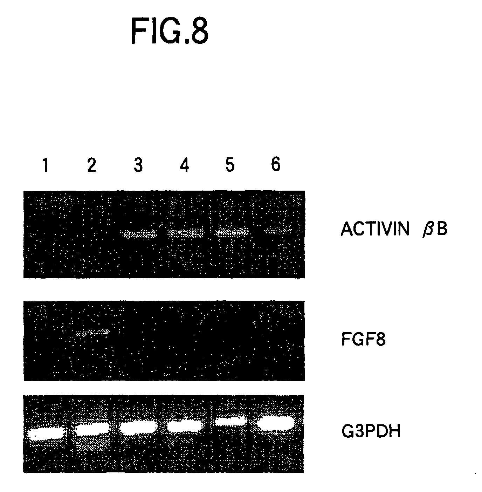

The results are shown in Fig. 8. Since all of the PCR bands of the control gene, G3PDH, were similar to one another, it was confirmed that amounts of mRNA in the androgen-dependent mouse breast cancer cell SC-3, and androgen-independent mouse breast cancer cells SC-4 and CADO21, used in the test were similar to one another.

In the androgen-independent mouse breast cancer cells SC-4 and CADO21, activin βB chain mRNA was detected independent of androgen stimulation or non-stimulation. On the other hand, activin βB chain mRNA was not detected independent of androgen stimulation or non-stimulation in the androgen-dependent mouse breast cancer cell SC-3. The FGF-8 mRNA was detected only in the androgen-stimulated androgen-dependent mouse breast cancer cell SC-3, and was not detected in the androgen-non-stimulated androgen-dependent mouse breast cancer cell SC-3, and androgen-independent mouse breast cancer cells SC-4 and CADO21.

It was revealed from the above results that androgen-dependent tumors produce FGF-8 under androgen stimulation but do not produce activin βB. Also, it was revealed that androgen-independent tumors do not produce FGF-8 but produce activin βB chain. This result shows that the cells lose FGF-8 productivity and acquire activin βB productivity when they are converted from androgen-dependent to independent. Accordingly, the conversion from androgen-dependent cells to independent cells can be diagnosed by detecting productivity of FGF-8 and activin βB chain by these cells.

### INDUSTRIAL APPLICABILITY

The present invention provide an inhibitor of the growth of an androgen-independent tumor useful for treating and diagnosing an androgen-independent tumor, an agent for treating and diagnosing an androgen-independent tumor, a method for diagnosing an androgen-independent tumor, and a method for screening a substance which inhibits the growth of an androgen-independent tumor cell.

Free text of the Sequence Listing
SEQ ID NO:1-Explanation of artificial sequence: synthetic DNA
SEQ ID NO:2-Explanation of artificial sequence: synthetic DNA
SEQ ID NO:3-Explanation of artificial sequence: synthetic DNA
SEQ ID NO:4-Explanation of artificial sequence: synthetic DNA

## Claims

1. An inhibitor of the growth of an androgen-independent tumor, comprising, as an active ingredient, a substance which inhibits the growth of an androgen-independent tumor.

2. The inhibitor of the growth of an androgen-independent tumor according to claim 1, wherein the substance which inhibits the growth of an androgen-independent tumor is at least one substance selected from the group consisting of a substance having inhibitory activity for activin and a substance having inhibitory activity for fibroblast growth factor-8 (FGF-8).

3. The inhibitor of the growth of an androgen-independent tumor according to claim 1 or 2, wherein the substance which inhibits the growth of an androgen-independent tumor is a substance having inhibitory activity for activin.

4. The inhibitor of the growth of an androgen-independent tumor according to claim 2 or 3, wherein the substance having inhibitory activity for activin is a substance selected from the group consisting of an anti-activin neutralizing antibody, an anti-activin receptor neutralizing antibody, an activin binding protein and an antisense DNA/RNA of activin.

5. The inhibitor of the growth of an androgen-independent tumor according to claim 4, wherein the activin binding protein is follistatin.

6. The inhibitor of the growth of an androgen-independent tumor according to claim 1 or 2, wherein the substance which inhibits the growth of an androgen-independent tumor is a substance having inhibitory activity for fibroblast growth factor-8 (FGF-8).

7. The inhibitor of the growth of an androgen-independent tumor according to claim 2 or 6, wherein the substance having inhibitory activity for fibroblast growth factor-8 (FGF-8) is an anti-FGF-8 neutralizing antibody.

8. An agent for treating an androgen-independent tumor, comprising, as an active ingredient, a substance which inhibits the growth of an androgen-independent tumor.

9. The agent for treating an androgen-independent tumor according to claim 8, wherein the substance which inhibits the growth of an androgen-independent tumor is at least one substance selected from the group consisting of a substance having inhibitory activity for activin and a substance having inhibitory activity for fibroblast growth factor-8 (FGF-8).

10. The agent for treating an androgen-independent tumor according to claim 8 or 9, wherein the substance which inhibits the growth of an androgen-independent tumor is a substance having inhibitory activity for activin.

11. The agent for treating an androgen-independent tumor according to claim 9 or 10, wherein the substance having inhibitory activity for activin is a substance selected from the group consisting of an anti-activin neutralizing antibody, an anti-activin receptor neutralizing antibody, an activin binding protein and an antisense DNA/RNA of activin.

12. The agent for treating an androgen-independent tumor according to claim 11, wherein the activin binding protein is follistatin.

13. The agent for treating an androgen-independent tumor according to claim 8 or 9, wherein the substance which inhibits the growth of an androgen-independent tumor is a substance having inhibitory activity for fibroblast growth factor-8 (FGF-8).

14. The agent for treating an androgen-independent tumor according to claim 7 or 13, wherein the substance having inhibitory activity for fibroblast growth factor-8 (FGF-8) is a substance selected from the group consisting of an anti-FGF-8 neutralizing antibody, an anti-FGF-8 receptor neutralizing antibody and an antisense DNA/RNA of FGF-8.

15. A method for detecting activin, comprising using a substance selected from the group consisting of an anti-activin antibody, an activin binding protein and a DNA/RNA coding for activin.

16. The method for detecting activin according to claim 15, wherein the activin binding protein is follistatin.

17. A method for determining activin, comprising using a substance selected from the group consisting of an anti-activin antibody, an activin binding protein and a DNA/RNA coding for activin.

18. The method for determining activin according to claim 16, wherein the activin binding protein is follistatin.

19. A method for diagnosing an androgen-independent tumor, comprising using a substance selected from the group consisting of an anti-activin antibody, an activin binding protein, a DNA/RNA coding for activin, an anti-FGF-8 antibody and a DNA/RNA coding for FGF-8.

20. The method for diagnosing an androgen-independent tumor according to claim 19, wherein the activin binding protein is follistatin.

21. A method for diagnosing the conversion of an androgen-dependent cell into an androgen-independent cell, comprising using a substance selected from the group consisting of an anti-activin antibody, an activin binding protein, a DNA/RNA coding for activin, an anti-FGF-8 antibody and a DNA/RNA coding for FGF-8.

22. The method according to (21), wherein the activin binding protein is follistatin.

23. An agent for diagnosing an androgen-independent tumor, comprising, as an active ingredient, a substance selected from the group consisting of an anti-activin antibody, an activin binding protein, a DNA/RNA coding for activin, an anti-FGF-8 antibody and a DNA/RNA coding for FGF-8.

24. The agent for diagnosing an androgen-independent tumor according to claim 23, wherein the activin binding protein is follistatin.

25. A method for screening an inhibitor of a growth factor for an androgen-independent tumor, comprising using a growth factor for an androgen-independent tumor.

26. The method for screening an inhibitor of a growth factor for an androgen-independent tumor according to claim 25, wherein the growth factor for an androgen-independent tumor is activin.

27. The method for screening an inhibitor of a growth factor for an androgen-independent tumor according to claim 25, wherein the growth factor for androgen-independent tumors is fibroblast growth factor-8 (FGF-8).


SEQUENCE LISTING
<110> Kyowa Hakko Kogyo Co., LTD

<120> Growth inhibitor of androgen-independent tumor

<130> 11204W01

<140>
<141>

<160> 4

<170> PatentIn Ver. 2.0

<210> 1
<211> 20
<212> DNA
<213> Artificial Sequence

<220>
<223> Description of Artificial Sequence:Synthetic DNA

<400> 1
cgagatcgca tccgcaaacg                                                    20

<210> 2
<211> 21
<212> DNA
<213> Artificial Sequence

<220>

<223>  Description of Artificial Sequence:Synthetic DNA

<400> 2
cactcctcca cgatccatgt t                                                21

<210> 3
<211> 21
<212> DNA
<213> Artificial Sequence

<220>
<223>  Description of Artificial Sequence:Synthetic DNA

<400> 3
tttacacagc atgtgaggga g                                                21

<210> 4
<211> 21
<212> DNA
<213> Artificial Sequence

<220>
<223>  Description of Artificial Sequence:Synthetic DNA

<400> 4
gtagttgagg aactcgaagc g                                                21